# EUROPEAN PATENT SPECIFICATION

(11) **EP 1 702 215 B1**
(45) Date of publication and mention of the grant of the patent: **12.03.2014**
(21) Application number: 05701808.7
(22) Date of filing: 07.01.2005
(51) Int. Cl.: B82Y 15/00

(54) **NANOPARTICLES AS AGENTS FOR IMAGING FINGER PRINTS**
NANOPARTIKEL ZUR BILDERERZEUGUNG VON FINGERABDRÜCKE
NANOPARTICULES UTILISEES EN TANT QU'AGENTS POUR L'IMAGERIE D'EMPREINTES DIGITALES

(30) Priority: 07.01.2004 GB 0400235
(43) Date of publication of application: 20.09.2006
(73) Proprietor: University of Sunderland, Sunderland, Tyne & Wear SR2 7EE (GB)
(72) Inventor: ROWELL, Frederick, John, Durham DH3 3LS (GB); THEAKER, Brenden, John, Stockton-on-Tees, Tees Valley TS20 1HG (GB)
(74) Representative: Selby, David Sean
(86) International application number: PCT/GB2005/000038
(87) International publication number: WO 2005/066632

(56) References cited:
- WO-A-03/083481
- WO-A-2004/063387
- US-A- 4 176 205
- US-A- 6 048 546
- US-A1- 2002 001 716
- US-B1- 6 194 213
- US-B1- 6 306 662
- US-B1- 6 495 352

## Description

This invention relates to a novel analytical method and to nanoparticles suitable for conducting such methods.

Fingerprints may be formed under a number of circumstances. The first involves transfer of a material such as blood or paint from the surface of the finger onto a surface. Alternatively the surface of the finger itself may cause an impression on a wet surface such as blood or paint, or leave an indentation in a plastic or malleable surface such as putty. In these and other examples, a clear print is often formed without the need to enhance or develop the print.

In a further alternative circumstance, contact of the finger on a surface may leave residues on the surface due to transfer of naturally occurring chemicals secreted from the skin. Such prints are termed latent fingerprints and they generally require treatment to render them visible. The chemical entities are derived from secretions from the eccrine and apocrine sweat glands and the sebaceous glands. Generally the major contributions are from the eccrine glands on the palms of the hand and the sebaceous glands from other areas of the skin. The major components of the secretions from the eccrine glands, are water (initially 98-99% but quickly lost through evaporation), anions including chloride, cations, amino acids, proteins urea, lactic acid and glucose - and from the sebaceous glands fatty acids, triglycerides, cholesterol, squalene, cholesteryl esters and wax esters.

To date a variety of strategies have been used to enhance the visibility of latent fingerprints. These include the use of specific methods targeted at a component within the print, and general methods which use a physical characteristic rather than a specific chemical interaction. Examples of the former include use of silver nitrate to form dark precipitates with chloride ions, use of ninhydrin which reacts with amino acids forming purple dyes, Amido black that sticks to proteins, iodine, Gentian violet and Sudan black that react with fatty acids. Examples of non-specific agents include super glue and dusting powders that stick to the hydrophobic, sebum-derived components of the prints.

US Patent No. 6,485,981 describes a method and apparatus for imaging and documenting fingerprints. A fluorescent dye brought in intimate proximity with the lipid residues of a latent fingerprint is caused to fluoresce on exposure to light energy. The resulting fluorescing image may be recorded photographically.

There is also a range of finely powdered dusting agents based on metals, carbon and lycopodium. Examples include fluorescent latent print powders that comprise different fluorescent dyes and can also have components that render them magnetic for ease of application.

Use of these reagents generally leads to prints that can be readily identified by their ridge patterns and their patterns of irregularity so that the overall characteristics can be used to identify the "owner" of the print. This may be problematic for prints deposited on porous surfaces or when only traces of the print are present.

We have now developed novel agents for developing latent fingerprints. These novel agents comprise fluorescent nanoparticles, generally formed as sol gel particles in the presence of fluorescent dye derivatives so that fluorescent nanoparticles are produced.

Thus, in the first aspect of the invention, we provide a fluorescent nanoparticle.

Preferably the nanoparticles of the invention sol gel-derived nanoparticles can be rendered fluorescent by entrapping biological macromolecules labelled with a variety of fluorescent reporter molecules. Examples of such combinations include Texas Red and fluorescein for proteins and ethidium bromide for DNA. The former dyes are covalently attached to the macromolecule and for the latter the dye molecule is intercalated with the DNA. Alternatively any nanoparticles having intrinsic fluorescence such as those derived from cadmium sulphide and cadmium selenide (and doped with rare earth atoms such as europium III salts) can be used.

The size of the nanoparticles may vary, however, it is preferred that the nanoparticles, which may be substantially spherical, may have a diameters of from 30 to 500 nm.

A variety of fluorescent dyes have been used based on entrapment of protein-dye conjugates within the nanoparticles during their preparation. Examples of dyes include Texas Red-labelled gelatin and porcine thyroglobulin, and fluorescein-labelled bovine serum albumin and gelatin.

Sol gels' processing generally comprises the formation of a dual phase material of a solid polymer matrix skeleton filled with a solvent through a sol gel transition. When the solvent is removed, the gel converts to a xerogel. Sol gels have been widely used as matrices in a variety of analytical systems, including for encapsulation of biological macromolecules such as proteins and enzymes or even whole cells.

In a biological application, the sol gel process comprises the preparation of an insoluble framework or cage in which the biological entity is entrapped or encapsulated.

Such particles can be modified by passive adsorption or via covalent attachment to coat their surfaces with hydrophobic molecules which facilitates their binding to hydrophobic deposits derived from latent fingerprints on surfaces. Examples of hydrophobic molecules include phosphatidylcholine and phosphatidylethanolamine although any hydrophobic molecule could in theory be used.

Sol gel-derived nanoparticles with a Texas Red-porcine thyroglobulin conjugate embedded within, have been shown to bind to latent fingerprints on surfaces as shown by the fluorescence of the Texas Red dye.

Since nanoparticles are much smaller than metal particles currently used to locate passive fingerprints it should be possible to discern greater details of the substructures of prints and to use this new detail to identify the originators of fingerprints with greater accuracy, even with incomplete prints. It should also be possible to use the high fluorescence intensity of the particles on the surface to improve the sensitivity of detection for such fingerprints.

We have modified the surfaces of these particles so that we can attach a variety of surface coatings to them. For finger print development the final coating is a lipophilic ("water-hating") bio-compatible chemical that preferentially binds to the sebum-derived components such as waxes, cholesterol and squalene. Examples of such lipophilic chemicals include phosphatidylcholine and phosphatidylethanolamine. To achieve this coating, we have either passively adsorbed the chemicals directly onto sol gel particles formed from TEMOS (tetramethyloxysilane) via electrostatic interactions, or have covalently coupled them to the particles using aminopropyloxysilane-derived sol gels. In the latter case attachment was via glutaraldehyde treatment followed by cyanoborohydride reduction and then washing with ethanolamine to reduce non-specific binding.

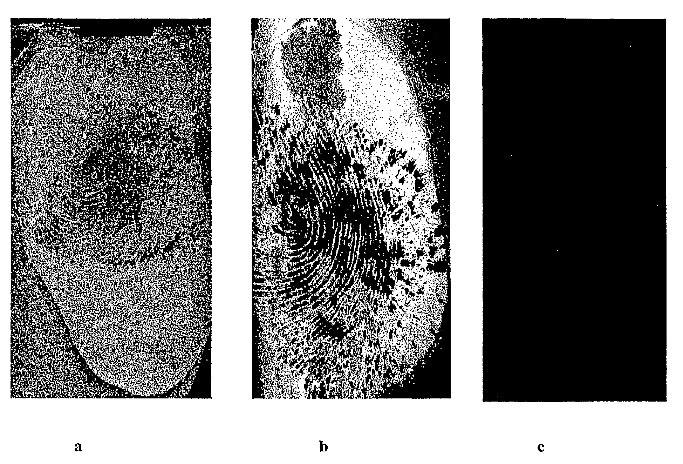

We have also prepared other sol gel-derived nanoparticles that are coated with hydrophilic ("water-loving") chemicals carrying either net negative or net positive charges. An example of the former includes uncoated nanoparticles whilst an example of the latter includes nanoparticles coated with polylysine. When we apply these hydrophilic particles to a finger print and scan the surface for fluorescence due to fluorescent dyes embedded within the particles, some fluorescence is seen but no development of the characteristic patterns is observed (Figure 1a): This scanning was performed at an excitation wavelength of 595 nm and an emission of 612 nm when Texas Red dye was used. In contrast when the lipophilic-coated particles are used, patterns of irregularity are clearly visible demonstrating selective binding to the sebum-derived components on the surface (Figure 1b). When no nanoparticles are added then no fluorescence is seen (Figure 1c) P104518GB.1

## Claims

1. A nanoparticle, which particle encapsulates a fluorescent material, **characterised in that** the nanoparticle comprises a fluorescent dye based on entrapment of a protein-dye conjugate or a DNA-dye conjugate within the nanoparticle, wherein the nanoparticle is derived from a sol gel.

2. A nanoparticle according to claim 1 **characterised in that** the nanoparticle is substantially spherical and has a diameter of from 30 to 500 nm.

3. A nanoparticle according to claim 1 **characterised in that** the dye is selected from Texas Red-labelled gelatin, porcine thyroglobulin, and fluorescein-labelled bovine serum albumin or gelatin.

4. A nanoparticle according to claim 1 **characterised in that** the surfaces of the particles are modified to enable them to be provided with a surface coating.

5. A nanoparticle according to claim 4 **characterised in that** the particles are capable of being modified by passive adsorption or via covalent attachment to coat their surfaces with hydrophobic molecules.

6. A nanoparticle according to claim 5 **characterised in that** the hydrophobic molecules are selected from phosphatidylcholine and phosphatidylethanolamine.

7. A nanoparticle according to claim 1 **characterised in that** the Sol gel-derived nanoparticles comprise a Texas Red-porcine thyroglobulin conjugate embedded within them.

8. A nanoparticle according to claim 1 **characterised in that** the particles comprise a high fluorescence intensity.

9. A nanoparticle according to claim 4 **characterised in that** the surface coating is lipophilic.

10. A nanoparticle according to claim 1 **characterised in that** the particle is adapted to bind to a sebum-derived component.

11. A nanoparticle according to claim 10 **characterised in that** the sebum derived component is selected from the group comprising waxes, cholesterol and squalene.

12. A nanoparticle according to claim 9 **characterised in that** the lipophilic coating is selected from phosphatidylcholine and phosphatidylethanolamine.

13. A nanoparticle according to claim 4 **characterised in that** the coating is passively adsorbed directly onto a sol gel particle.

14. A nanoparticle according to claim 1 **characterised in that** the particles are formed from TEMOS (tetramethyloxysilane).

15. A nanoparticle according to claim 1 **characterised in that** the particles comprise aminopropyloxysilane-derived sol gels.

16. A nanoparticle according to claim 15 **characterised in that** the preparation of the particles included a glutaraldehyde treatment.

17. A nanoparticle according to claim 16 **characterised in that** the glutaraldehyde treatment was followed by cyanoborohydride reduction.

18. A nanoparticle according to claim 17 **characterised in that** the cyanoborohydride reduction was followed by an ethanolamine wash.

19. A nanoparticle according to claim 1 **characterised in that** the particles are uncoated nanoparticles and carry either a net negative or a net positive charge.

20. A method of detecting prints (e.g. fingerprints) which comprises the use of a nanoparticle according to any of claims 1 to 19.

21. A method according to claim 20 which comprises determining details of fingerprint substructures.

22. A method according to claim 20 or 21 **characterised in that** a scanning is performed at an excitation wavelength of 595 nm.

## Patentansprüche

1. Nanopartikel, wobei der Partikel ein fluoreszentes Material verkapselt, **dadurch gekennzeichnet, dass** der Nanopartikel einen fluoreszenten Farbstoff umfasst, der auf dem Einbau eines Protein-Farbstoff-Konjugats oder eines DNA-Farbstoff-Konjugats in den Nanopartikel beruht, wobei der Nanopartikel aus einem Solgel abgeleitet ist.

2. Nanopartikel nach Anspruch 1, **dadurch gekennzeichnet, dass** der Nanopartikel im Wesentlichen sphärisch ist und einen Durchmesser von 30 bis 500 nm aufweist.

3. Nanopartikel nach Anspruch 1, **dadurch gekennzeichnet, dass** der Farbstoff ausgewählt ist aus Texas-Rot markierter Gelatine, Schweinethyroglobulin und Fluorescein markiertem Rinderserumalbumin oder Gelatine.

4. Nanopartikel nach Anspruch 1, **dadurch gekennzeichnet, dass** die Oberflächen der Partikel modifiziert sind, um sie in die Lage zu versetzen, mit einer Oberflächenbeschichtung versehen zu werden.

5. Nanopartikel nach Anspruch 4, **dadurch gekennzeichnet, dass** die Partikel durch passive Adsorption oder durch kovalente Anlagerung modifiziert werden können, um ihre Oberflächen mit hydrophoben Molekülen zu beschichten.

6. Nanopartikel nach Anspruch 5, **dadurch gekennzeichnet, dass** die hydrophoben Moleküle ausgewählt sind aus Phosphatidylcholin und Phosphatidylethanolamin.

7. Nanopartikel nach Anspruch 1, **dadurch gekennzeichnet, dass** die von Solgel abgeleiteten Partikel ein Texas-Rot-Schweinethyroglobulin-Konjugat umfassen, das darin eingebettet ist.

8. Nanopartikel nach Anspruch 1, **dadurch gekennzeichnet, dass** die Partikel eine hohe Fluoreszenzintensität umfassen.

9. Nanopartikel nach Anspruch 4, **dadurch gekennzeichnet, dass** die Oberflächenbeschichtung lipophil ist.

10. Nanopartikel nach Anspruch 1, **dadurch gekennzeichnet, dass** der Partikel bearbeitet ist, um an eine von Sebum abgeleitete Komponente zu binden.

11. Nanopartikel nach Anspruch 10, **dadurch gekennzeichnet, dass** die von Sebum abgeleitete Komponente ausgewählt ist aus der Gruppe, die Wachse, Cholesterin und Squalen umfasst.

12. Nanopartikel nach Anspruch 9, **dadurch gekennzeichnet, dass** die lipophile Beschichtung ausgewählt ist aus Phosphatidylcholin und Phosphatidylethanolamin.

13. Nanopartikel nach Anspruch 4, **dadurch gekennzeichnet, dass** die Beschichtung passiv direkt auf einen Solgelpartikel adsorbiert wird.

14. Nanopartikel nach Anspruch 1, **dadurch gekennzeichnet, dass** die Partikel aus TEMOS (Tetramethyloxysilan) gebildet werden.

15. Nanopartikel nach Anspruch 1, **dadurch gekennzeichnet, dass** die Partikel von Aminopropyloxysilan abgeleitete Solgele umfassen.

16. Nanopartikel nach Anspruch 15, **dadurch gekennzeichnet, dass** die Herstellung der Partikel eine Glutaraldehydbehandlung einschloss.

17. Nanopartikel nach Anspruch 16, **dadurch gekennzeichnet, dass** die Glutaraldehydbehandlung von einer Cyanborhydridreduktion gefolgt wurde.

18. Nanopartikel nach Anspruch 17, **dadurch gekennzeichnet, dass** die Cyanborhydridreduktion von einer Ethanolaminwaschung gefolgt wurde.

19. Nanopartikel nach Anspruch 1, **dadurch gekennzeichnet, dass** die Partikel unbeschichtete Nanopartikel sind und entweder eine negative oder positive Nettoladung tragen.

20. Verfahren zum Nachweis von Abdrücken (z.B. Fingerabdrücken), das die Verwendung eines Nanopartikels nach einem der Ansprüche 1 bis 19 umfasst.

21. Verfahren nach Anspruch 20, das die Bestimmung von Details von Unterstrukturen von Fingerabdrücken umfasst.

22. Verfahren nach Anspruch 20 oder 21, **dadurch gekennzeichnet, dass** ein Abscannen bei einer Anregungswellenlänge von 595 nm durchgeführt wird.

## Revendications

1. Nanoparticule, laquelle particule encapsule un matériau fluorescent, **caractérisée en ce que** la nanoparticule comprend un colorant fluorescent basé sur le piégeage d'un conjugué de protéine marqué avec un colorant ou d'un conjugué d'ADN marqué avec un colorant dans la nanoparticule, dans lequel la nanoparticule est dérivée d'un sol-gel.

2. Nanoparticule selon la revendication 1 **caractérisée en ce que** la nanoparticule est substantiellement sphérique et a un diamètre allant de 30 à 500 nm.

3. Nanoparticule selon la revendication 1 **caractérisée en ce que** le colorant est sélectionné à partir de la gélatine marquée au Texas Red, la thyroglobuline porcine, et la gélatine ou l'albumine de sérum bovin marquée à la fluorescéine.

4. Nanoparticule selon la revendication 1 **caractérisée en ce que** les surfaces des particules sont modifiées pour leur permettre d'être complétées avec une surface d'enrobage.

5. Nanoparticule selon la revendication 4 **caractérisée en ce que** les particules sont capables d'être modifiées par adsorption passive ou par l'intermédiaire d'une liaison covalente pour revêtir leur surface avec des molécules hydrophobes.

6. Nanoparticule selon la revendication 5 **caractérisée en ce que** les molécules hydrophobes sont sélectionnées à partir de la phosphatidylcholine et de la phosphatidyléthanolamine.

7. Nanoparticule selon la revendication 1 **caractérisée en ce que** les nanoparticules dérivées du sol-gel comprennent un conjugué de thyroglobuline porcine marqué au Texas Red.

8. Nanoparticule selon la revendication 1 **caractérisée en ce que** les particules ont une intensité de fluorescence élevée.

9. Nanoparticule selon la revendication 4 **caractérisée en ce que** la surface d'enrobage est lipophile.

10. Nanoparticule selon la revendication 1 **caractérisée en ce que** la particule est adaptée pour se lier à un composant dérivé du sébum.

11. Nanoparticule selon la revendication 10 **caractérisée en ce que** le composant dérivé du sébum est sélectionné du groupe comprenant des cires, du cholestérol et du squalène.

12. Nanoparticule selon la revendication 9 **caractérisée en ce que** l'enrobage lipophile est sélectionné entre la phosphatidylcholine et la phosphatidyléthanolamine.

13. Nanoparticule selon la revendication 4 **caractérisée en ce que** l'enrobage est passivement adsorbé directement sur une particule sol-gel.

14. Nanoparticule selon la revendication 1 **caractérisée en ce que** les particules sont formées à partir du TEMOS (tétraméthyloxysilane).

15. Nanoparticule selon la revendication 1 **caractérisée en ce que** les particules comprennent des sol-gels dérivés de l'aminopropyloxysilane.

16. Nanoparticule selon la revendication 15 **caractérisée en ce que** la préparation des particules incluait un traitement au glutaraldéhyde.

17. Nanoparticule selon la revendication 16 **caractérisée en ce que** le traitement au glutaraldéhyde a été suivi d'une réduction au cyanoborohydrure.

18. Nanoparticule selon la revendication 17 **caractérisée en ce que** la réduction au cyanoborohydrure a été suivie par un lavage à l'éthanolamine.

19. Nanoparticule selon la revendication 1 **caractérisée en ce que** les particules sont des nanoparticules non enrobées et produisent une charge nette négative ou une charge nette positive.

20. Procédé de détection d'empreintes (exemple : empreintes digitales) comprenant l'utilisation d'une nanoparticule selon l'une quelconque des revendications 1 à 19.

21. Procédé selon la revendication 20 qui comprend la détermination des détails des sous-structures de l'empreinte digitale.

22. Procédé selon la revendication 20 ou 21 **caractérisé en ce qu'**un scannage est réalisé à une longueur d'onde d'excitation de 595 nm.
